# EUROPEAN PATENT APPLICATION

(11) **EP 4 715 939 A1**
(43) Date of publication of application: **25.03.2026**
(21) Application number: 24935748.4
(22) Date of filing: 19.12.2024
(51) Int. Cl.: H01M 10/0567, H01M 10/0568

(54) **ELECTROLYTE ADDITIVE, ELECTROLYTE, AND BATTERY**

(30) Priority: 19.04.2024 CN 202410475714
(71) Applicant: Guangzhou Tinci Materials Technology Co., Ltd., Guangzhou, Guangdong 510760 (CN)
(72) Inventor: REN, Li, Guangzhou, Guangdong 510670 (CN); LI, Shuailong, Guangzhou, Guangdong 510670 (CN); XIE, Tian, Guangzhou, Guangdong 510670 (CN)
(74) Representative: Plougmann Vingtoft a/s
(86) International application number: PCT/CN2024/140650
(87) International publication number: WO 2025/218220

(57) **Abstract**

The present disclosure provides an electrolyte additive, an electrolyte, and a battery. The electrolyte additive comprises: a first additive and a second additive. The structure of the first additive is as represented by formula 1, and the second additive comprises a trinitrile substance.

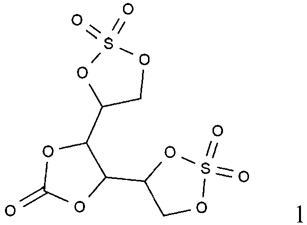

## Description

### CROSS-REFERENCE TO RELATED APPLICATIONS

This application claims priority to the Chinese patent application No. 202410475714.1, filed with the China National Intellectual Property Administration on April 19, 2024, and entitled "ELECTROLYTE ADDITIVE, ELECTROLYTE, AND BATTERY", which is incorporated herein by reference in its entirety.

### FIELD

The present disclosure relates to the technical field of electrolytes, and specifically, to an electrolyte additive, an electrolyte, and a battery.

### BACKGROUND

With the rapid development of markets such as pure electric vehicles, smart homes, traction tools, and smart transportation, the requirements of consumers for the performance of batteries are constantly increasing. Metal ion batteries, such as lithium-ion batteries and sodium-ion batteries, are widely used in consumer electronics and energy storage and power batteries due to their advantages such as high energy density, long cycle life, and low self-discharge. Typically, a battery includes a positive electrode plate, a negative electrode plate, a separator, and an electrolyte. The electrolyte includes a solvent, an electrolyte salt, and an electrolyte additive. By adding the electrolyte additive to the electrolyte, the performance of the battery such as rapid charge and discharge, cycle life, and storage stability can be improved to a certain extent. However, the current electrolyte additives are not sufficient to improve the performance of the battery to meet the demands of the consumers and still need to be further improved.

### SUMMARY

In a first aspect of the present disclosure, the present disclosure provides an electrolyte additive. The electrolyte additive includes a first additive and a second additive. The first additive has a structure represented by Formula 1, 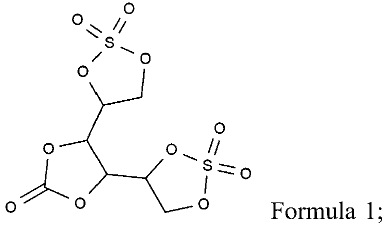 and the second additive includes trinitrile compounds. Therefore, the high-temperature cycle performance of the battery using the electrolyte additive can be effectively improved.

In some embodiments, a mass fraction of the first additive in the electrolyte additive is w₁; a mass fraction of the second additive in the electrolyte additive is w₂; and w₁/w₂ ranges from 0.9 to 1.1. Therefore, it is facilitated to further improve the high-temperature cycle performance of the battery.

In some embodiments, the second additive includes at least one of 1,3,6-hexanetricarbonitrile, 1,3,5-pentanetricarbonitrile, or 1,2,3-tris(2-cyanoethoxy)propane. Therefore, the electrical performance of the electrolyte can be improved.

In some embodiments, the electrolyte additive further includes a third additive. The third additive includes at least one of hexamethylene diisocyanate or trimethylsilyl isothiocyanate. Therefore, it is facilitated to further improve the cycle life of the battery.

In some embodiments, the electrolyte additive further includes a fourth additive. The fourth additive includes at least one of fluoroethylene carbonate, difluoroethylene carbonate, 1,3-propylene sultone, 1,3-propane sultone, ethylene sulfate, or methylene methanedisulfonate. Therefore, it is facilitated to form an SEI film on the surface of the negative electrode active material.

In a second aspect of the present disclosure, the present disclosure provides an electrolyte. The electrolyte includes the aforementioned electrolyte additive. Therefore, the electrolyte has all the characteristics and advantages of the aforementioned electrolyte additive, which will not be described in detail here.

In some embodiments, a mass fraction of the first additive in the electrolyte ranges from 0.1% to 2%. Therefore, the cycle life of the battery can be improved, and the internal resistance of the battery can be reduced.

In some embodiments, a mass fraction of the second additive in the electrolyte ranges from 0.1% to 2%. Therefore, the electrical performance of the battery can be improved.

In some embodiments, a mass fraction of the third additive in the electrolyte ranges from 0.1% to 0.3%. Therefore, it is facilitated to further improve the cycle life of the battery.

In some embodiments, a mass fraction of the fourth additive in the electrolyte ranges from 0.1% to 10%. Therefore, it is facilitated to improve the cycle performance of the battery.

In some embodiments, the electrolyte further includes an electrolyte salt. The electrolyte salt includes at least one of LiPF₆, LiAsF₆, LiClO₄, LiBF₄, LiB(C₂O₄)₂, LiBF₂C₂O₄, LiN(SO₂F)₂, LiN(SO₂CF₃)₂, LiPO₂F₂, LiPF₂(C₂O₄)₂, LiPF₄C₂O₄, or NaFP₆. Therefore, the ionic conductivity of the electrolyte can be improved.

In some embodiments, a mass fraction of the electrolyte salt in the electrolyte ranges from 8 wt % to 20 wt %. Therefore, the electrolyte can have both high ionic conductivity and low cost.

In a third aspect of the present disclosure, the present disclosure provides a battery. The battery includes the aforementioned electrolyte additive and/or the aforementioned electrolyte. Therefore, the battery has all the characteristics and advantages of the aforementioned electrolyte additive and electrolyte, which will not be described in detail here.

In some embodiments, the battery further includes a positive electrode plate. The positive electrode plate includes a positive electrode current collector and a positive electrode active material layer located at least on one side of the positive electrode current collector; the positive electrode active material layer includes a positive electrode active material; and a mass fraction of element manganese in the positive electrode active material is greater than or equal to 25%. Therefore, the cost of the positive electrode active material can be reduced, and the discharge voltage platform of the battery can be improved.

In some embodiments, the positive electrode active material satisfies a general formula of LiMnₓFe₁₋ₓPO₄, where 0.25<x<1; and/or a general formula of LiNi_{1-y}Mn_{y}O, where 0.25<y<1; and/or a general formula of Na_{z}MnO₂, where 0.5<z<1.

### DETAILED DESCRIPTION

Unless otherwise defined, all technical and scientific terms used in the present disclosure have the same meanings as commonly understood by those skilled in the art to which the present disclosure belongs. The terms used in the present disclosure are only for the purpose of describing specific embodiments and are not intended to limit the present disclosure. Unless otherwise specified, the numerical values of the various parameters mentioned in the present disclosure can be measured using various measurement methods commonly used in the art (for example, they can be measured according to the methods given in the embodiments of the present disclosure).

The terms "include" and "have" in the specification and claims of the present disclosure and any variations thereof are open-ended expressions, that is, including the contents specified in the present disclosure but not excluding other contents.

In the description of the present disclosure, regardless of whether the words "about" or "approximately" are used, all numbers disclosed herein are approximate values. The value of each number may vary by less than 10% or by amounts deemed reasonable by those skilled in the art, such as 1%, 2%, 3%, 4%, or 5%.

In the description of the present disclosure, the terms "first" and "second" are used merely for descriptive purposes and should not be understood as indicating or implying relative importance or implicitly specifying the quantity of the indicated technical features. "First feature" and "second feature" may include one or more of the features.

In the description of the present disclosure, "A and/or B" may include any one of the case of A alone, the case of B alone, or the case of A and B, where A and B are only used for examples, and may be any technical feature connected by "and/or" in the present disclosure.

Manganese is a common element on Earth with abundant resource reserves, making manganese-based positive electrode active materials an alternative solution to alleviate resource pressure. The manganese-based positive electrode active materials have also become an important research direction in the field of metal ion batteries due to their advantages such as high gram capacity, low cost, and good thermal stability. Taking lithium-ion batteries as an example, during the charge and discharge process of the battery, Mn⁴⁺ in the manganese-based positive electrode active material will undergo a disproportionation reaction and dissolve into the electrolyte in the form of Mn²⁺. The manganese ions dissolved in the electrolyte will migrate and deposit on the surface of the negative electrode active material, destroying the SEI film (Solid Electrolyte Interface) on the surface of the negative electrode active material. After the SEI film ruptures, the exposed negative electrode active material will react with the electrolyte, resulting in the decomposition of lithium salts and the generation of HF. In this way, the positive electrode material is corroded, thereby further leading to the dissolution of Mn. This process is repeated, not only causing irreversible loss of lithium ions, but also leading to the consumption of the electrolyte, thereby significantly declining the cycle performance of the battery. In addition, when the battery is under high-temperature conditions, the positive electrode active material may undergo lattice collapse or structural transformation. In this way, manganese ions are released from the structure, and the movement rate of manganese ions increases significantly, which accelerates the speed at which manganese ions deintercalate from the crystal structure and significantly increases the chemical reaction rate of manganese dissolution.

In the present disclosure, a first additive and a second additive are used in combination. The second additive is a trinitrile compound. The trinitrile additive has a stable molecular structure and superior oxidation resistance. The cyano group of the trinitrile compound can react with the manganese ions dissolved from the positive electrode active material to form a complex, thereby reducing the damage of manganese ions to the SEI film on the surface of the negative electrode active material. Furthermore, the first additive has a symmetrical structure, and there is an intermolecular force between the first additive and the second additive. As a result, the triangular structure of the second additive (i.e., the triangular structure defined by the linkages between adjacent cyano groups in the second additive) and the symmetrical structure of the first additive can jointly realize the chelation of the manganese ions. Specifically, the cyano group of the second additive is used to complex the manganese ions, and the interaction between the first additive and the second additive is used to define the manganese ions in the chelation structure formed by the first additive and the second additive. In this way, the complexing effect of the electrolyte additive on the free manganese ions can be effectively enhanced, the migration of manganese ions to the surface of the negative electrode active material can be effectively reduced, and the side reaction of manganese ions with other substances in the electrolyte can be reduced. Moreover, the positive and negative electrodes of the battery can be facilitated to obtain better interface stability, and the cycle performance of the battery, in particular the high-temperature cycle performance, can be improved.

In a first aspect of the present disclosure, the present disclosure provides an electrolyte additive. The electrolyte additive includes a first additive and a second additive. The first additive has a structure represented by Formula 1, 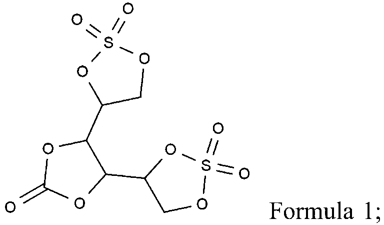 and the second additive includes trinitrile compounds. Therefore, by utilizing the synergistic effect of the first additive and the second additive, transition metal ions dissolved from the positive electrode active material, such as manganese ions, can be effectively captured. In this way, the deposition of the transition metal ions on the surface of the negative electrode active material can be effectively reduced, and the structural stability of the SEI film on the surface of the negative electrode active material can be improved, thereby effectively improving the cycle performance of the battery using the electrolyte additive, in particular the high-temperature cycle performance.

In some embodiments, the first additive can act as a film-forming additive to participate in the formation of the SEI film on the surface of the negative electrode active material. Thus, the first additive can not only capture transition metal ions together with the second additive in the electrolyte, but also capture transition metal ions in the SEI film on the surface of the negative electrode active material, thereby reducing the damage of the transition metal ions to the negative electrode active material.

In some embodiments, a mass fraction of the first additive in the electrolyte additive is w₁; a mass fraction of the second additive in the electrolyte additive is w₂; and w₁/w₂ ranges from 0.9 to 1.1. Therefore, it is facilitated to further improve the high-temperature cycle performance of the battery.

As an example, w₁/w₂ may be 0.9, 0.95, 1.0, 1.05, or 1.1.

The cyano group in the second additive can react with the transition metal ions dissolved from the positive electrode active material, such as manganese ions, to form a complex, reducing the damage of manganese ions to the SEI film on the surface of the negative electrode active material. However, when the content of the cyano group in the electrolyte additive is too high, the internal resistance of the battery will be increased, which in turn causes the deterioration of the performance of the battery. There is a weak intermolecular force between the sulfur-oxygen double bond in the first additive and the transition metal ions, which can bind the transition metal ions to a certain extent. Using the first additive and the second additive satisfying the aforementioned ratio and partially replacing the second additive with the first additive, the deposition of free transition metal ions on the surface of the negative electrode active material can be further reduced by utilizing the synergistic effect of the first additive and the second additive while decreasing the amount of the second additive and lowering the internal resistance of the battery.

In some embodiments, the second additive may include at least one of 1,3,6-hexanetricarbonitrile, 1,3,5-pentanetricarbonitrile, or 1,2,3-tris(2-cyanoethoxy)propane. Therefore, the electrical performance of the battery can be improved.

When the second additive includes the aforementioned substances, the second additive has a moderate carbon chain length, a moderate molecular weight, a high cyano density, a strong complexing ability for transition metal ions, and excellent electrochemical performance.

In some embodiments, the electrolyte additive further includes a third additive. The third additive includes at least one of hexamethylene diisocyanate or trimethylsilyl isothiocyanate. Therefore, it is facilitated to further improve the cycle life of the battery.

The water in the battery will react irreversibly with the lithium salt (such as lithium hexafluorophosphate) in the electrolyte inside the battery to generate hydrofluoric acid (HF) and other harmful byproducts. Trace acidic substances (such as hydrofluoric acid HF) in the electrolyte will corrode the positive electrode active material, accelerate the dissolution of transition metal ions in the positive electrode active material, and further reduce the cycle performance of the battery. The addition of the third additive can undergo physical adsorption or chemical reaction with water and acidic substances in the battery, thereby reducing the acidic substances in the electrolyte, lowering its content to a lower level, and slowing down the dissolution of transition metal ions in the positive electrode active material.

In some embodiments, the electrolyte additive further includes a fourth additive. The fourth additive includes at least one of fluoroethylene carbonate, difluoroethylene carbonate, 1,3-propylene sultone, 1,3-propane sultone, ethylene sulfate, or methylene methanedisulfonate. Therefore, it is facilitated to improve the cycle performance of the battery.

The addition of the fourth additive can form an SEI (Solid Electrolyte Interface) on the surface of the negative electrode active material and a CEI (Cathode Electrolyte Interface) on the surface of the positive electrode active material. SEI and CEI form films on the surfaces of negative electrode active materials and positive electrode active materials, respectively, which can effectively alleviate the problem of side reactions occurring after direct contact between active materials and electrolyte.

In a second aspect of the present disclosure, the present disclosure provides an electrolyte. The electrolyte includes the aforementioned electrolyte additive. Therefore, the electrolyte has all the characteristics and advantages of the aforementioned electrolyte additive, which will not be described in detail here.

In some embodiments, a mass fraction of the first additive in the electrolyte ranges from 0.1% to 2%. Therefore, the cycle life of the battery can be improved, and the internal resistance of the battery can be reduced.

As an example, the mass fraction of the first additive in the electrolyte may be 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, or 2.0%.

In some embodiments, a mass fraction of the second additive in the electrolyte ranges from 0.1% to 2%. Therefore, the electrical performance of the battery can be improved.

As an example, the mass fraction of the second additive in the electrolyte may be 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, or 2.0%.

When the mass fractions of the first additive and the second additive are within the aforementioned ranges, the mass fraction w₁ of the first additive and the mass fraction w₂ of the second additive can satisfy that w₁/w₂ ranges from 0.9 to 1.1. Partially replacing the second additive with the first additive, the deposition of free transition metal ions on the surface of the negative electrode active material can be effectively reduced by utilizing the synergistic effect of the first additive and the second additive while decreasing the amount of the second additive and lowering the internal resistance of the battery, thereby improving the cycle performance of the battery.

In some embodiments, a mass fraction of the third additive in the electrolyte ranges from 0.1% to 0.3%. Therefore, it is facilitated to further improve the cycle life of the battery.

As an example, the mass fraction of the third additive in the electrolyte may be 0.1%, 0.15%, 0.2%, 0.25%, or 0.3%.

When the mass fraction of the third additive is within the aforementioned range, the addition of a relatively small amount of the water-scavenging and acid-inhibiting agent can effectively slow down the dissolution of transition metal ions in the positive electrode active material, thereby reducing the manufacturing cost.

In some embodiments, a mass fraction of the fourth additive in the electrolyte ranges from 0.1% to 10%. Therefore, it is facilitated to improve the cycle performance of the battery.

As an example, the mass fraction of the fourth additive in the electrolyte may be 0.1%, 0.5%, 1.0%, 1.5%, 2.0%, 2.5%, 3%, 3.5%, 4.0%, 4.5%, 5.0%, 5.5%, 6.0%, 6.5%, 7.0%, 7.5%, 8.0%, 8.5%, 9.0%, 9.5%, or 10.0%.

When the mass fraction of the fourth additive is within the aforementioned range, it helps to form a uniform and dense SEI (Solid Electrolyte Interface) on the surface of the negative electrode active material and a uniform and dense CEI (Cathode Electrolyte Interface) on the surface of the positive electrode active material, thereby alleviating the problem of side reactions occurring after direct contact between the active material and the electrolyte.

In some embodiments, the electrolyte further includes an electrolyte salt. The electrolyte salt includes at least one of LiPF₆, LiAsF₆, LiClO₄, LiBF₄, LiB(C₂O₄)₂, LiBF₂C₂O₄, LiN(SO₂F)₂, LiN(SO₂CF₃)₂, LiPO₂F₂, LiPF₂(C₂O₄)₂, LiPF₄C₂O₄, or NaFP₆. Therefore, the ionic conductivity of the electrolyte can be improved.

In some embodiments, a mass fraction of the electrolyte salt in the electrolyte ranges from 8 wt % to 20 wt %. Therefore, the electrolyte can have both high ionic conductivity and low cost.

The electrolyte salt accounts for a high proportion of the cost of the electrolyte. When the mass fraction of the electrolyte salt in the electrolyte is within the above range, the electrolyte salt can be fully dissolved in the solvent, and thus the electrolyte can have both high ionic conductivity and low manufacturing cost.

In some embodiments, the solvent of the electrolyte includes at least one of ethylene carbonate, propylene carbonate, dimethyl carbonate, diethyl carbonate, methyl ethyl carbonate, γ-butyrolactone, methyl acetate, ethyl acetate, propyl acetate, butyl acetate, ethyl propionate, propyl propionate, or butyl propionate.

The solvent in the electrolyte is an important carrier for ion transport. When the electrolyte salt is dissolved, the electrolyte can have a higher ionic conductivity. Then, by selecting the above solvent, the cycle life, charge and discharge rate, high-temperature performance, low-temperature performance, and energy density of the battery can be improved.

In some embodiments, a mass fraction of the solvent in the electrolyte may be 50 wt% to 80 wt%. Therefore, it is facilitated to fully dissolve the electrolyte salt and improve the conductivity of the electrolyte.

In a third aspect of the present disclosure, the present disclosure provides a battery. The battery includes the aforementioned electrolyte additive and/or the aforementioned electrolyte. Therefore, the battery has all the characteristics and advantages of the aforementioned electrolyte additive and electrolyte, which will not be described in detail here.

Typically, a battery includes a positive electrode plate, a negative electrode plate, an electrolyte, and a separator. During the charge and discharge process of the battery, active ions intercalate and deintercalate back and forth between the positive electrode plate and the negative electrode plate. The electrolyte conducts ions between the positive electrode plate and the negative electrode plate. The separator is set between the positive electrode plate and the negative electrode plate, mainly to prevent the positive and negative electrodes from short-circuiting, while allowing ions to pass through.

In some embodiments, the battery further includes a positive electrode plate. The positive electrode plate includes a positive electrode current collector and a positive electrode active material layer located at least on one side of the positive electrode current collector; the positive electrode active material layer includes a positive electrode active material; and a mass fraction of element manganese in the positive electrode active material is greater than or equal to 25%. Therefore, the cost of the positive electrode active material can be reduced, and the discharge voltage platform of the battery can be improved.

When the mass fraction of element manganese in the positive electrode active material is within the aforementioned range, the positive electrode active material can have a higher gram capacity, which can effectively improve the energy density of the battery. In addition, the charge cut-off voltage of the battery can reach 4.35V. The battery can have a higher discharge platform and show good cycle stability under the conventional voltage window. Moreover, because manganese reserves are relatively abundant and the price thereof is relatively low worldwide, compared with rare and expensive metals such as cobalt and nickel, the use of the aforementioned positive electrode active material can help to reduce costs and reduce dependence on limited resources. Further, the use of manganese-rich positive electrode active materials with cobalt-free or low-cobalt and low-nickel formulas is also beneficial to reducing the environmental impact during the production of the battery and improving environmental protection.

In the present disclosure, the first additive and the second additive are used in combination. The first additive has a symmetrical structure, and there is an intermolecular force between the first additive and the second additive. As a result, the triangular structure of the second additive (i.e., the triangular structure defined by the linkages between adjacent cyano groups in the second additive) and the symmetrical structure of the first additive can jointly realize the chelation of the manganese ions. In this way, the complexing effect of the electrolyte additive on the free manganese ions can be effectively enhanced, the migration of manganese ions to the surface of the negative electrode active material can be effectively reduced, and the side reaction of manganese ions with other substances in the electrolyte can be reduced. Moreover, the positive and negative electrodes of the battery can be facilitated to obtain better interface stability, and the cycle performance of the battery, in particular the high-temperature cycle performance, can be improved.

In some embodiments, the positive electrode active material satisfies the general formula LiMnₓFe₁₋ₓPO₄, where 0.25<x<1.

In some embodiments, the positive electrode active material satisfies the general formula LiNi_{1-y}Mn_{y}O₄, where 0.25<y<1.

In some embodiments, the positive electrode active material satisfies the general formula Na_{z}MnO₂, where 0.5<z<1.

In some embodiments, the positive electrode current collector may include a metal foil or a composite current collector. For example, the metal foil may be an aluminum foil. The composite current collector may include a polymer substrate and a metal layer formed on at least one side surface of the polymer substrate. The composite current collector can be formed by forming a metal material (aluminum, aluminum alloy, nickel, nickel alloy, titanium, titanium alloy, silver and silver alloy, etc.) on a polymer substrate material (such as polypropylene (PP), polyethylene terephthalate (PET), polybutylene terephthalate (PBT), polystyrene (PS), polyethylene (PE), etc.).

In some embodiments, the positive electrode active material layer may further optionally include a binder. As an example, the binder may include at least one of polyvinylidene fluoride (PVDF), polytetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, or fluorine-containing acrylate resin.

In some embodiments, the positive electrode active material layer may further optionally include a conductive agent. For example, the conductive agent may include at least one of superconducting carbon, acetylene black, carbon black, Ketjen black, carbon dots, carbon nanotubes, graphene, or carbon nanofibers.

In some embodiments, the negative electrode plate includes a negative electrode current collector and a negative electrode active material layer disposed on at least one surface of the negative electrode current collector. The negative electrode active material layer includes a negative electrode active material. The negative electrode active material may include at least one of artificial graphite, natural graphite, soft carbon, hard carbon, mesocarbon microbeads, silicon-based materials, tin-based materials, or lithium titanate.

In some embodiments, the negative electrode current collector may contain a metal foil or a composite current collector. For example, a copper foil can be used as the metal foil. The composite current collector may include a polymer material base layer and a metal layer formed on at least one surface of the polymer material base layer. The composite current collector can be formed by forming a metal material (copper, copper alloy, nickel, nickel alloy, titanium, titanium alloy, silver and silver alloy, etc.) on a polymer material substrate (such as polypropylene (PP), polyethylene terephthalate (PET), polybutylene terephthalate (PBT), polystyrene (PS), polyethylene (PE), etc.).

In some embodiments, the negative electrode active material layer may further include a binder, a conductive agent, and other auxiliary agents. For example, the binder may include at least one of styrene-butadiene rubber (SBR), polyacrylic acid (PAA), sodium polyacrylate (PAAS), polyacrylamide (PAM), polyvinyl alcohol (PVA), sodium alginate (SA), polymethacrylic acid (PMAA), or carboxymethyl chitosan (CMCS). The conductive agent may include at least one of superconducting carbon, acetylene black, carbon black, Ketjen black, carbon dots, single-walled carbon nanotubes, graphene, or carbon nanofibers. The auxiliary agent may include a thickener, such as sodium carboxymethyl cellulose (CMC-Na) and the like.

The present disclosure has no particular limitation on the type of the separator, and any porous separator with good chemical stability and mechanical stability can be selected. For example, the material of the separator may include at least one of glass fiber, non-woven fabric, polyethylene, polypropylene, or polyvinylidene fluoride. The separator may be a single-layer film or a multi-layer composite film.

The battery of the present disclosure may include a battery cell form, a battery module form, and a battery pack form.

The present disclosure has no particular limitation on the shape of the battery cell, which may be cylindrical, cube, or any other shape. In some embodiments, the outer packaging of the battery cell may be a hard shell, such as a hard plastic shell, an aluminum shell, a steel shell, etc. The outer packaging of the battery cell may also be a soft package, such as a pouch-type soft package. The material of the soft package may be plastic, such as polypropylene, polybutylene terephthalate, and polybutylene succinate.

In some embodiments, the battery cells can be assembled into a battery module. The number of the battery cells contained in a battery module may be one or more. The specific number may be selected by those skilled in the art based on the application and capacity of the battery module.

In some embodiments, the battery modules can also be assembled into a battery pack. The number of the battery modules contained in the battery pack may be one or more. The specific number can be selected by those skilled in the art based on the application and capacity of the battery pack.

The battery cells, the battery modules, and the battery packs can be used as power sources for the electrical devices or as energy storage units for the electrical devices. The electrical devices may include, but are not limited to, mobile devices (such as mobile phones, laptops, etc.), electric vehicles (such as pure electric vehicles, hybrid electric vehicles, plug-in hybrid electric vehicles, electric bicycles, electric scooters, electric golf carts, electric trucks, etc.), electric trains, ships and satellites, energy storage systems, etc. For the electrical device, the battery, the battery module, or the battery pack can be selected according to usage requirements.

The scheme of the present disclosure will be described below through specific examples. It should be noted that the following examples are only used to illustrate the present disclosure and should not be regarded as limiting the scope of the present disclosure. Where specific techniques or conditions are not specified in the examples, they are performed according to techniques or conditions described in the literature in the art or according to the product description. The reagents or instruments used are conventional products that can be obtained commercially without indicating the manufacturer.

### Example 1

Preparation of a positive electrode plate: a positive electrode active material LiMn_{0.6}Fe_{0.4}O₄, a binder polyvinylidene fluoride (PVDF), a conductive agent carbon black, and a conductive agent carbon nanotubes in a weight ratio of 95: 2.5: 2: 0.5 were mixed evenly, and N-methylpyrrolidone (NMP) was added thereto. The mixture was stirred using a vacuum mixer until the mixed system became a positive electrode slurry with uniform fluidity. The positive electrode slurry was evenly coated on a positive electrode current collector aluminum foil with a coating amount of 35 g/m². The system was dried at 85°C, and then cold pressed, trimmed, cut, and striped. After stripping, the system was dried at 85°C under vacuum conditions for 4 hours. Finally, a positive electrode plate was obtained after welding tabs.

Preparation of a negative electrode plate: a negative electrode active material graphite, a conductive agent carbon black, a thickener sodium carboxymethyl cellulose (CMC-Na), and a binder styrene-butadiene rubber in a weight ratio of 95: 1.5: 1: 2.5 were mixed, and deionized water was added thereto. A negative electrode slurry was obtained using a vacuum mixer. The negative electrode slurry was evenly coated on a negative electrode collector copper foil with a coating amount of 20 g/m². The system was dried at 85°C, and then cold pressed, trimmed, cut, and striped. After stripping, the system was dried at 85°C under vacuum conditions for 4 hours. Finally, a negative electrode plate was obtained after welding tabs.

Preparation of an electrolyte: in an argon-filled glove box (moisture <10 ppm, oxygen <1 ppm), solvents ethylene carbonate, propylene carbonate, and diethyl carbonate in a mass ratio of 3: 5: 2 were uniformly mixed. Thoroughly dried lithium hexafluorophosphate was quickly added to the mixed solvent to make the mass fraction of lithium hexafluorophosphate in the electrolyte 12.5 wt%. The following electrolyte additives were also added, where the mass fractions were the mass fractions of the corresponding substances based on the electrolyte: a first additive, 0.5 wt% of the compound represented by Formula 1; a second additive, 0.5 wt% of 1,3,6-hexanetrinitrile; and a fourth additive, 5 wt% of fluoroethylene carbonate.

Preparation of a separator: a polyethylene separator with a thickness of 8 µm was selected.

Preparation of a lithium-ion battery: the positive electrode plate, the negative electrode plate, and the separator prepared according to the above process were laminated to form a lithium-ion battery with a thickness of 4.7 mm, a width of 55 mm, and a length of 60 mm. The battery was baked under vacuum at 75°C for 10 hours, and the electrolyte prepared above was injected thereto. After standing for 24 hours, the battery was placed in a 45°C environment, a pressure of 3 kg was applied, and the battery was charged to 4.0 V at 0.1C (160 mA) and then stood for 2 days (to fully activate the battery) to obtain a battery.

The differences between other Examples and Comparative Examples and Example 1 were specifically shown in Table 1. Specifically, compared with Example 1, Examples 2, 3, and 13 used different trinitrile compounds; Examples 4 and 5 used different positive electrode active materials; Examples 6, 7, and 8 added a third additive; Examples 9, 10, 11, and 12 had different mass fractions of the first additive and the second additive; and Example 14 had a different positive electrode active material, and lithium hexafluorophosphate was replaced with sodium hexafluorophosphate therein. The first additive was not added in Comparative Example 1, and the second additive was not added in Comparative Example 2.

**[Table 1]**

| Serial number | Positive electrode active material | Mass fraction of first additive in electrolyte / wt% | Second additive in electrolyte | Mass fraction of second additive in electrolyte / wt% | Third additive and its mass fraction in the electrolyte / wt% |
|---|---|---|---|---|---|
| Example 1 | LiMn_{0.6}Fe_{0.4}PO₄ | 0.5 | 1,3,6-hexanetricarbonitrile | 0.5 | / |
| Example 2 | LiMn_{0.6}Fe_{0.4}PO₄ | 0.5 | Pentanetricarbonitrile | 0.5 | / |
| Example 3 | LiMn_{0.6}Fe_{0.4}PO₄ | 0.5 | Tris(2-cyanoethoxy)propane | 0.5 | / |
| Example 4 | LiNi_{0.5}Mn_{0.5}O₄ | 0.5 | 1,3,6-hexanetricarbonitrile | 0.5 | / |
| Example 5 | Li₂MnO₃ | 0.5 | 1,3,6-hexanetricarbonitrile | 0.5 | / |
| Example 6 | LiMn_{0.6}Fe_{0.4}PO₄ | 0.5 | 1,3,6-hexanetricarbonitrile | 0.5 | Hexamethylene diisocyanate, 0.1 |
| Example 7 | LiMn_{0.6}Fe_{0.4}PO₄ | 0.5 | 1,3,6-hexanetricarbonitrile | 0.5 | Hexamethylene diisocyanate, 0.2 |
| Example 8 | LiMn_{0.6}Fe_{0.4}PO₄ | 0.5 | 1,3,6-hexanetricarbonitrile | 0.5 | Hexamethylene diisocyanate, 0.3 |
| Example 9 | LiMn_{0.6}Fe_{0.4}PO₄ | 0.1 | 1,3,6-hexanetricarbonitrile | 0.1 | / |
| Example 10 | LiMn_{0.6}Fe_{0.4}PO₄ | 2 | 1,3,6-hexanetricarbonitrile | 2 | / |
| Example 11 | LiMn_{0.6}Fe_{0.4}PO₄ | 0.9 | 1,3,6-hexanetricarbonitrile | 1 | / |
| Example 12 | LiMn_{0.6}Fe_{0.4}PO₄ | 1.1 | 1,3,6-hexanetricarbonitrile | 1 | / |
| Example 13 | LiMn_{0.6}Fe_{0.4}PO₄ | 0.5 | 1,3,5-benzenetricarbonitrile | 0.5 | / |
| Example 14 | Na_{0.6}MnO₂ | 0.5 | 1,3,6-hexanetricarbonitrile | 0.5 | / |
| Example 15 | LiMn_{0.25}Fe_{0.75}PO₄ | 0.59 | 1,3,6-hexanetricarbonitrile | 0.45 | / |
| Example 16 | LiMn_{0.8}Fe_{0.2}PO₄ | 0.5 | 1,3,6-hexanetricarbonitrile | 0.5 | / |
| Example 17 | LiMn_{0.6}Fe_{0.4}PO₄ | 0.5 | 1,3,6-hexanetricarbonitrile, pentanetricarbonitrile | 1,3,6-hexanetricarbonitrile, 0.5; pentanetricarbonitrile, 0.3 | / |
| Comparative Example 1 | LiMn_{0.6}Fe_{0.4}PO₄ | / | 1,3,6-hexanetricarbonitrile | 0.5 | / |
| Comparative Example 2 | LiMn_{0.6}Fe_{0.4}PO₄ | 0.5 | / | / | / |

The batteries in the above Examples and Comparative Examples were tested as follows. The test results are shown in Table 2.

High-temperature cycle performance test: at 45°C, the lithium-ion battery was charged and discharged for 500 times at a current of 0.5C. Capacity retention rate (%) = (500th discharge capacity/1st discharge capacity) × 100%.

Manganese dissolution test: for the negative electrode plate, the battery after 500 cycles was disassembled. The negative electrode plate was taken out, rinsed with 30 g of DMC (dimethyl carbonate) for 3 times, and then soaked with 20 g of water. After the negative electrode active material was detached from the copper foil, the negative electrode active material was filtered and dried. 0.5 g of negative electrode active material was weighed, 10 mL of nitric acid and 10 mL of sulfuric acid were added thereto, and the mixture was heated until the sample was dissolved (the solution was milky white) and then cooled. 10 mL of hydrochloric acid was added, and the system was heated until the salts were dissolved (the solution was clear). The solution was cooled and filtered using filter paper with a pore size of 50 µm. The filter paper and the beaker were rinsed. The filtrate and the rinsing water were transferred to a 250 mL volumetric flask. Finally, an ICP test was performed, and the manganese content x₁ was recorded; for the electrolyte, the battery after 500 cycles was disassembled. The battery cell was taken out and centrifuged at high speed in a centrifuge. 0.5 g of electrolyte was taken, 10 mL of nitric acid and 10 mL of sulfuric acid were slowly added thereto, and the mixture was heated until the sample was dissolved (the solution was milky white) and then cooled. 10 mL of hydrochloric acid was added, and the system was heated until the salts were dissolved (the solution was clear). The solution was cooled and filtered using filter paper with a pore size of 50 µm. The filter paper and the beaker were rinsed. The filtrate and the rinsing water were transferred to a 250 mL volumetric flask. Finally, an ICP test was performed, and the manganese content x₂ was recorded. The amount of manganese dissolution in the electrolyte + negative electrode plate was x₁ + x₂.

**[Table 2]**

| Serial number | Capacity retention rate after 500 cycles/% | Manganese dissolution in electrolyte + negative electrode plate /ppm |
|---|---|---|
| Example 1 | 91.33 | 28.43 |
| Example 2 | 90.86 | 27.95 |
| Example 3 | 91.18 | 28.22 |
| Example 4 | 89.80 | 35.27 |
| Example 5 | 90.39 | 31.51 |
| Example 6 | 92.61 | 26.59 |
| Example 7 | 92.37 | 25.98 |
| Example 8 | 90.95 | 25.12 |
| Example 9 | 87.30 | 30.60 |
| Example 10 | 89.24 | 28.15 |
| Example 11 | 87.69 | 28.40 |
| Example 12 | 88.21 | 27.84 |
| Example 13 | 79.80 | 87.60 |
| Example 14 | 87.57 | 30.45 |
| Example 15 | 91.06 | 27.56 |
| Example 16 | 90.29 | 32.62 |
| Example 17 | 91.88 | 28.08 |
| Comparative Example 1 | 70.20 | 110.20 |
| Comparative Example 2 | 72.50 | 109.50 |

As can be seen from Table 2, by comparing Examples 1 to 5 and Comparative Examples 1 to 2, the second additive in the present disclosure has a good synergistic effect with the first additive, significantly reducing the dissolution of manganese ions in the battery, effectively reducing the rupture and reorganization of the SEI film caused by the deposition of manganese ions on the surface of the SEI film, and effectively improving the cycle performance of the battery.

It can be seen from Examples 6 to 8 that the added third additive easily reacts with water and acid, thereby reducing the generation of hydrofluoric acid. Further, the corrosion of HF on the positive electrode active material is reduced, the dissolution of manganese ions in the positive electrode active material is effectively inhibited, and the cycle performance of the battery is improved.

It should be noted that the present disclosure is not limited to the above-mentioned embodiments. The above embodiments are merely examples, and any embodiments having substantially the same structure and effect as the technical idea within the scope of the technical solution of the present disclosure are all included in the technical scope of the present disclosure. Furthermore, other embodiments obtained by adding various modifications that can be conceived by those skilled in the art to the embodiments and combining some of the constituent elements of the embodiments are also included in the scope of the present disclosure without departing from the spirit of the present disclosure.

## Claims

1. An electrolyte additive, comprising:
a first additive, wherein the first additive has a structure represented by Formula 1, 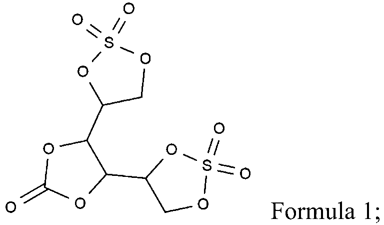 and
a second additive, wherein the second additive comprises trinitrile compounds.

2. The electrolyte additive according to claim 1, wherein:
a mass fraction of the first additive in the electrolyte additive is w₁;
a mass fraction of the second additive in the electrolyte additive is w₂; and
w₁/w₂ ranges from 0.9 to 1.1.

3. The electrolyte additive according to claim 1 or 2, wherein the second additive comprises at least one of 1,3,6-hexanetricarbonitrile, 1,3,5-pentanetricarbonitrile, or 1,2,3-tris(2-cyanoethoxy)propane.

4. The electrolyte additive according to any one of claims 1 to 3, further comprising a third additive, wherein the third additive comprises at least one of hexamethylene diisocyanate or trimethylsilyl isothiocyanate.

5. The electrolyte additive according to any one of claims 1 to 4, further comprising a fourth additive, wherein the fourth additive comprises at least one of fluoroethylene carbonate, difluoroethylene carbonate, 1,3-propylene sultone, 1,3-propane sultone, ethylene sulfate, or methylene methanedisulfonate.

6. An electrolyte, comprising the electrolyte additive according to any one of claims 1 to 5.

7. The electrolyte according to claim 6, wherein a mass fraction of the first additive in the electrolyte ranges from 0.1% to 2%.

8. The electrolyte according to claim 6 or 7, wherein a mass fraction of the second additive in the electrolyte ranges from 0.1% to 2%.

9. The electrolyte according to any one of claims 6 to 8, wherein a mass fraction of the third additive in the electrolyte ranges from 0.1% to 0.3%.

10. The electrolyte according to any one of claims 6 to 9, wherein a mass fraction of the fourth additive in the electrolyte ranges from 0.1% to 10%.

11. The electrolyte according to any one of claims 6 to 10, further comprising an electrolyte salt, wherein the electrolyte salt comprises at least one of LiPF₆, LiAsF₆, LiClO₄, LiBF₄, LiB(C₂O₄)₂, LiBF₂C₂O₄, LiN(SO₂F)₂, LiN(SO₂CF₃)₂, LiPO₂F₂, LiPF₂(C₂O₄)₂, LiPF₄C₂O₄, or NaFP₆.

12. The electrolyte according to claim 11, wherein a mass fraction of the electrolyte salt in the electrolyte ranges from 8 wt% to 20 wt%.

13. A battery, comprising:
the electrolyte additive according to any one of claims 1 to 5; and/or
the electrolyte according to any one of claims 6 to 12.

14. The battery according to claim 13, further comprising a positive electrode plate, wherein the positive electrode plate comprises a positive electrode current collector and a positive electrode active material layer located at least on one side of the positive electrode current collector, the positive electrode active material layer comprising a positive electrode active material, a mass fraction of element manganese in the positive electrode active material being greater than or equal to 25%.

15. The battery according to claim 14, wherein the positive electrode active material satisfies:
a general formula of LiMnₓFe₁₋ₓPO₄, where 0.25<x<1; and/or
a general formula of LiNi_{1-y}Mn_{y}O, where 0.25<y<1; and/or
a general formula of Na_{z}MnO₂, where 0.5<z<1.
